Europäisches Patentamt

European Patent Office

Office européen des brevets

(11) Publication number: **0 462 290 A1**

# EUROPEAN PATENT APPLICATION
## published in accordance with Art. 158(3) EPC

(21) Application number: 91901902.6

(22) Date of filing: 09.01.91

(86) International application number:
PCT/JP91/00007

(87) International publication number:
WO 91/10429 (25.07.91 91/17)

(51) Int. Cl.5: **A61K 31/20, A23L 1/30**

(30) Priority: 09.01.90 JP 1001/90

(43) Date of publication of application:
27.12.91 Bulletin 91/52

(84) Designated Contracting States:
CH DE FR GB IT LI NL SE

(71) Applicant: KABUSHIKI KAISYA ADVANCE
5-7, Nihonbashi Kobuna-cho
Chuo-ku Tokyo 103(JP)

(72) Inventor: ISHIHARA, Kazuoki
4-4-12-1302, Goushitsu Sagamihara
Sagamihara-shi Kanagawa 229(JP)
Inventor: AKASAKA, Akinobu
Sanraifuhigashihashimoto 202
3-23-32, Higashihashimoto
Sagamihara-shi Kanagawa 229(JP)

(74) Representative: Rinuy, Santarelli
14, avenue de la Grande Armée
F-75017 Paris(FR)

(54) LIPID ABSORPTION INHIBITING COMPOSITION.

(57) A lipid absorption inhibiting composition containing as an active ingredient a $C_{18}$ to $C_{22}$ fatty acid containing one unsaturated double bond in its molecule.

TECHNICAL FIELD

The present invention relates to a novel lipid absorption inhibitory composition.

BACKGROUND ART

The formation of micell by bile acid is important to the digestive absorption process of a lipid, particularly a triglyceride, and among bile acids, conjugated bile acid has a particularly high micell forming ability for a lipid.

Further, some enterobacteria have a disconjugating ability by which conjugated bile acid is hydrolyzed to form a free bile acid (e.g., Enterococcus, Streptococcus, Lactobacillus, Bifidobacferium, and Clostridium, etc.), and when the disconjugating action in the upper part of the small intestine is too strong, the digestive absorption of fat will become poor and this will cause diarrhea. Furthermore, it has been reported that domestic animals, etc., with a high feces disconjugating ability do not gain weight. Free bile acid, different to conjugated bile acid, is not actively transported during reabsorption, and therefore, free bile acid is easily discharged and thus a disassimilation of cholesterol is accelerated. Nowadays however, the intake of fat and other nutrients is often excessive, and the bile acid disconjugating action of enterobacteria may serve to make the intake of nutrients adequate.

DISCLOSURE OF INVENTION

Accordingly, an object of the present invention is to develop a substance which intensifies the bile acid disconjugating ability of enterobacteria.

Other objects and specific features of the present invention will be apparent from the following description.

In accordance with the present invention, there is provided a lipid absorption inhibitory composition comprising a fatty acid having 18 to 22 carbon atoms and one unsaturated double bond in the molecule as the active ingredient.

BEST MODE OF CARRYING OUT THE INVENTION

The constitution and the effect of the invention will be described in detail as follows.

FATTY ACIDS

Specific examples of fatty acids used in the present invention include petroselinic acid, petroselaidic acid, elaidic acid, cisvaccenic acid, transvaccenic acid, 9-eicosenic acid (cadlenic acid), 11-eicosenic acid (gondoic acid), cetrenic acid, and erucic acid. These fatty acids can be used alone or as a mixture of two or more thereof.

According to the present invention, these fatty acids can be formulated in an appropriate carrier, e.g., hydroxypropylmethyl cellulose phtalate or sein. (preferable content: 0.5% to 3% by weight), and further, if necessary, a perfume or dye, etc., can be formulated as desired.

METHOD OF USE AND DOSAGE

The lipid absorption inhibitory composition according to the present invention is usually effective at a dose of about 0.1 mg to 5 mg/kg (body weight)/day when administered orally, and since these fatty acids are also food components, they are substantially nontoxic even upon oral administration. As the dosage form, in addition to conventional oral dosage forms such as tablets and capsules, etc., the composition can be utilized in the form of a food auxiliary agent or food composition, as a functional food component.

PREPARATION OF MIXTURE OF FATTY ACIDS

The fatty acids according to the present invention can be prepared industrially advantageously as the oxygen decomposed or alkali hydrolyzed products of lipids derived from microorganisms such as lactic acid bacteria, rice bran, and wheat bran. The methods of preparation are exemplified below.

(1) Preparation of Microorganism Lipids

First, after culturing the bacteria in, for example, a GAM medium (Nissui Seiyaku), cells were collected by centrifugation (8000 x g), washed twice with physiological saline, and lyophilized or dried by heating, extracted with hexane, ethanol or dichloromethane:methanol (2:1) followed by a removal of the solvent by a rotary evaporator, to obtain lipids.

As the yeast, a commercially available baker's yeast was dried and treated in the same way with the organic solvent mentioned above, whereby lipids were obtained.

Further, lipids can be similarly obtained from rice bran and wheat bran.

(2) Decomposition (hydrolysis) treatment of lipids

(i) A 1 g amount of the lipids obtained as described above is suspended in, for example, 100 ml of a 0.15 M phosphate buffer (pH 8.0) containing 1% polyvinyl alcohol, 1% pancreatin, lipase or esterase is added thereto, and the treatment is carried out of 40°C for 3 hours. In place of the lipids obtained according to the method (i), microorganism cells or rice bran or wheat bran can be directly treated with the enzyme. Next, the product is extracted twice with the same volume of hexane, the hexane layer is recovered, and after removal of the hexane, for example, a lipid decomposed product is obtained.

(ii) To 1 g of the above lipid is added 100 ml of a 4N KOH solution, and after treatment at 60°C for 30 minutes while stirring, the treated mixture is made acidic with HCl, extracted with the same volume of hexane or ether, the extracted layer removed, followed by the removal of the organic solvent, and a lipid decomposed product is obtained.

BILE ACID DISCONJUGATING ABILITY MEASURING METHOD

Enterobacteria (Enterococcus, Lactobacillus, Bifidobacterium, Bacteroides) were cultured over night (37°C, initial living cells $10^5$/ml) in a GYP medium (glucose 1%, yeast extract 0.2%, peptone 0.2%, monopotassium phosphate 0.3%, dipotassium phosphate 0.3%, adjusted to pH 7 with sodium hydroxide) or GAM (Nissui Seiyaku) medium, cells were collected by centrifugation, washed twice with physiological saline, suspended in a 50 mM phosphate buffer (pH 6.0) of the same volume as the culture broth, and 1/20 volume of 20 mM conjugated bile acid (taurochenodeoxycholic acid or glycochenodeoxycholic acid) added, followed by a reaction at 37°C for 0, 6, 12 minutes. To complete the reaction, the same volume of 20% trichloroacetic acid solution is added and the mixture stirred. The mixture is then centrifuged at 10000 x g, the concentration of tree amino acid in the supernatant is measured by ninhydrin reaction, and the bile acid disconjugation rate is defined as the amino acid concentration increase rate. For an examination of fatty acids or rice bran lipid decomposed products, these lipids are added to 0 to 4% in the above liquid medium, and the bile acid disconjugating activity of the cultured cells is measured and represented as the ratio relative to the activity when no addition is made 1.

EXAMPLES

The present invention is described in more detail with reference to Examples, which in no way limit the scope of the present invention.

(i) Bile acid disconjugating ability elevation ratio[*] (fold) of E. faecium FERM BP-296

First, 0.01% of each the respective fatty acids was added to the medium, and the microorganism then inoculated, and thereafter, the bile acid disconjugating activity of the microorganism cells after 16 to 20 hours was measured.

[*] The activity of the microorganism cultured in a medium without an addition of fatty acid is made 1.

|  | Glycine conjugate | Taurine conjugate |
|---|---|---|
| Petroseric acid | 6.0 | 9.5 |
| Petroselaidic acid | 6.3 | 9.4 |
| Elaidic acid | 5.4 | 9.9 |
| Cisvaccenic acid | 5.7 | 7.2 |
| Transvaccenic acid | 5.7 | 6.8 |

Also, other $C_{18}$ to $C_{22}$ monoenic acids exhibited activities substantially equal to those shown above.

(ii) Elevation ratio of disconjugating abilities of various lactic acid bacteria with addition of rice bran lipid decomposed product

|  | Taurine conjugate | Glycine conjugate |
|---|---|---|
| E. faecium Co816 | 9.3 | 8.0 |
| S.bovis Co825 | 6.2 | - |
| Lactobacillus Co829 | 6.8 | 7.5 |
| Lactobacillus Ro123b | 1.8 | 2.0 |
| Bac. fragilis | 1.8 | 1.6 |
| Bif. breve AD0055 | 8.2 | 3.2 |

(note) The rice bran lipid decomposed product was added to the medium to 0.4%.

(iii) Concentration dependency of the rice bran lipid decomposed product on elevation of bile acid disconjugating activity elevation *1

| Concentration of lipid decomposed product | T*2 | G*3 |
|---|---|---|
| 0% | 1 | 1 |
| 0.05 | 4.3 | 3.8 |
| 0.1 | 9.5 | 5.7 |
| 0.2 | 9.0 | 6.8 |
| 0.3 | - | - |
| 0.4 | 9.3 | 8.0 |

*1: relative value (value when 0% is made 1)

*2: T ... activity elevation relative to taurine conjugate

*3: G ... glycine

E. faecium Co was cultured in GYP medium as the basal medium.

(iv) Bile acid disconjugating activity elevation action of lipids derived from microorganisms

| Origin of decomposed lipid (derived from) | T | G |
|---|---|---|
| L. reuteri AD0002 | 7.1 | 2.0 |
| E. faecalis AD1001 | 4.2 | 1.8 |
| S. salivarius AD10015 | 4.3 | 1.6 |
| B. breve AD0055 | 6.2 | 1.9 |
| Wheat bran | 7.7 | 2.8 |

(Note) Lipid added to medium at 0.1%.

(v) Influence of rice bran lipid decomposed product administration on rat

| | Bile acid disconjugating activity (Unit/g) | |
|---|---|---|
| | Jejunum | Ileum |
| Administered group | 0.40 | 0.42 |
| Control group | 0.28 | 0.18 |

(Note) Wister KY rats 10 weeks old were used. The control group was fed ordinary food, and the administered group (average value of n = 3) was fed food to which was added 0.4% of the rice bran lipid decomposed product, feeding was continued for 2 weeks, and thereafter, the rats were dissected and examined.

UTILIZATION IN INDUSTRY

As apparent from the above description, even though the lipid absorption inhibitory composition containing $C_{18}$ - $C_{22}$ fatty acids is derived from food components, and therefore is substantially orally nontoxic, it has an effective lipid absorption inhibitory activity and is particularly useful as a functional food component.

**Claims**

1. A lipid absorption inhibitory composition comprising a fatty acid having 18 to 22 carbon atoms and one unsaturated double bond in the molecule as the active ingredient.

2. A lipid absorption inhibitory composition, wherein said fatty acid is at least one selected from the group consisting of petroselinic acid, petroselaidic acid, elaidic acid, cisvaccenic acid, transvaccenic acid, 9-eicosenic acid (cadlenic acid), 11-eicosenic acid (gondoic acid), cetrenic acid and erucic acid.

# INTERNATIONAL SEARCH REPORT

International Application No PCT/JP91/00007

## I. CLASSIFICATION OF SUBJECT MATTER (if several classification symbols apply, indicate all) 6

According to International Patent Classification (IPC) or to both National Classification and IPC

Int. Cl$^5$  A61K31/20, A23L1/30

## II. FIELDS SEARCHED

### Minimum Documentation Searched 7

| Classification System | Classification Symbols |
|---|---|
| IPC | A61K31/20, A23L1/30 |

### Documentation Searched other than Minimum Documentation to the Extent that such Documents are Included in the Fields Searched 8

## III. DOCUMENTS CONSIDERED TO BE RELEVANT 9

| Category * | Citation of Document, 11 with indication, where appropriate, of the relevant passages 12 | Relevant to Claim No. 13 |
|---|---|---|
| A | JP, A, 55-92316 (Zaidan Hojin Kagakuhin Kensa Kyokai), July 12, 1980 (12. 07. 80), Claim (Family: none) | 1-2 |
| A | JP, A, 1-128919 (Snow Brand Milk Products Co., Ltd.), May 22, 1989 (22. 05. 89), Page 1 (Family: none) | 1-2 |
| P | JP, A, 2-286621 (Mitsubishi Kasei Corp.), November 26, 1990 (26. 11. 90), Claim (Family: none) | 1-2 |

* Special categories of cited documents: 10

"A" document defining the general state of the art which is not considered to be of particular relevance

"E" earlier document but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step

"Y" document of particular relevance; the claimed invention cannot be considered to involve an inventive step when the document is combined with one or more other such documents, such combination being obvious to a person skilled in the art

"&" document member of the same patent family

## IV. CERTIFICATION

| Date of the Actual Completion of the International Search | Date of Mailing of this International Search Report |
|---|---|
| March 27, 1991 (27. 03. 91) | April 8, 1991 (08. 04. 91) |

| International Searching Authority | Signature of Authorized Officer |
|---|---|
| Japanese Patent Office | |

Form PCT/ISA/210 (second sheet) (January 1985)